(19) Europäisches Patentamt
European Patent Office
Office européen des brevets

(11) **EP 1 830 637 B1**

(12) **EUROPEAN PATENT SPECIFICATION**

(45) Date of publication and mention
of the grant of the patent:
**29.09.2010 Bulletin 2010/39**

(51) Int Cl.:
***A01N 31/02*** *(2006.01)*

(21) Application number: **05858574.6**

(22) Date of filing: **05.12.2005**

(86) International application number:
**PCT/US2005/043910**

(87) International publication number:
**WO 2007/044032 (19.04.2007 Gazette 2007/16)**

(54) **COMPOSITIONS HAVING A HIGH ANTIVIRAL AND ANTIBACTERIAL EFFICACY**

ZUSAMMENSETZUNGEN MIT HOHER ANTIVIRALER UND ANTIBAKTERIELLER WIRKUNG

PRÉPARATIONS À EFFET ANTIVIRAL ET ANTIBACTÉRIEN IMPORTANT

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **09.12.2004 US 634464 P**

(43) Date of publication of application:
**12.09.2007 Bulletin 2007/37**

(73) Proprietor: **The Dial Corporation
Scottsdale, AZ 85254-2199 (US)**

(72) Inventors:
• **FULS, Janice, Lynn
Fountain Hills, AZ 85268-2964 (US)**
• **TAYLOR, Timothy, J.
Phoenix, AZ 85013 (US)**
• **FOX, Priscilla, S.
Phoenix, AZ 85028 (US)**
• **RODGERS, Nancy, Day
Chandler, AZ 85248 (US)**
• **TOWNER, Harry, Ernest
Scottsdale, AZ 85260 (US)**
• **DALTON, James
Scottsdale, AZ 85254 (US)**

(74) Representative: **Semrau, Markus
Henkel AG & Co. KGaA
Patente (FJP)
40191 Düsseldorf (DE)**

(56) References cited:
**EP-A1- 0 707 794      WO-A-01/28339
WO-A-2006/062857   GB-A- 1 126 953
GB-A- 2 391 810      US-A- 4 647 458
US-A- 4 767 788      US-A- 6 034 133
US-A- 6 136 771**

• **DATABASE WPI Week 199202 Derwent
Publications Ltd., London, GB; AN 1989-136366
XP002429055 & SE 466 111 B (ASTRA AB) 16
December 1991 (1991-12-16)**

**Description**

## CROSS REFERENCE TO RELATED APPLICATIONS

This application claims the benefit of U.S. provisional patent application Serial No. 60/634,464, filed December 9, 2004, and U.S. provisional patent application Serial No. 60/634,483, filed December 9, 2004.

## FIELD OF THE INVENTION

The present invention relates to antimicrobial compositions having a rapid and a persistent antiviral effectiveness, and a rapid, broad-spectrum antibacterial effectiveness. More particularly, the present invention relates to antimicrobial compositions comprising (a) a disinfecting alcohol and (b) an organic acid. The combination of (a) and (b) can provide a synergistic reduction in Gram negative and Gram positive bacteria and/or can synergistically inactivate or destroy viruses, such as rhinoviruses and rotaviruses, based on the log P (water-octanol partition coefficient) of the organic acid. The compositions provide a substantial reduction, e.g., greater than 99%, in nonenveloped viral populations, and in Gram negative and Gram positive bacterial populations, within one minute.

## BACKGROUND OF THE INVENTION

Human health is impacted by a variety of microbes encountered on a daily basis. In particular, contact with various microbes in the environment can lead to an illness, possibly severe, in mammals. For example, microbial contamination can lead to a variety of illnesses, including, but not limited to, food poisoning, a streptococcal infection, anthrax (cutaneous), athlete's foot, cold sores, conjunctivitis ("plink eye"), coxsackie-virus (hand-foot-mouth disease), croup, diphtheria (cutaneous), ebolic hemorrhagic fever, and impetigo.

It is known that washing body parts (e.g., hand washing) and hard surfaces (e.g., countertops and sinks) can significantly decrease the population of microorganisms, including pathogens. Therefore, cleaning skin and other animate and inanimate surfaces to reduce microbial populations is a first defense in removing such pathogens from these surfaces, and thereby minimizing the risk of infection.

Viruses are a category of pathogens of primary concern. Viral infections are among the greatest causes of human morbidity, with an estimated 60% or more of all episodes of human illness in developed countries resulting from a viral infection. In addition, viruses infect virtually every organism in nature, with high virus infection rates occurring among all mammals, including humans, pets, livestock, and zoo specimens.

Viruses exhibit an extensive diversity in structure and life cycle. A detailed description of virus families, their structure, life cycles, and modes of viral infection is discussed in Fundamental Virology, 4th Ed., Eds. Knipe & Howley, Lippincott Williams & Wilkins, Philadelphia, PA, 2001.

Simply stated, virus particles are intrinsic obligate parasites, and have evolved to transfer genetic material between cells and encode sufficient information to ensure their propagation. In a most basic form, a virus consists of a small segment of nucleic acid encased in a simple protein shell. The broadest distinction between viruses is the enveloped and nonenveloped viruses, i.e., those that do or do not contain, respectively, a lipid-bilayer membrane.

Viruses propagate only within living cells. The principal obstacle encountered by a virus is gaining entry into the cell, which is protected by a cell membrane of thickness comparable to the size of the virus In order to penetrate a cell, a virus first must become attached to the cell surface. Much of the specificity of a virus for a certain type of cell lies in its ability to attach to the surface of that specific cell. Durable contact is important for the virus to infect the host cell, and the ability of the virus and the cell surface to interact is a property of both the virus and the host cell. The fusion of viral and host-cell membranes allows the intact viral particle, or, in certain cases, only its infectious nucleic acid to enter the cell. Therefore, in order to control a viral infection, it is important to rapidly kill a virus that contacts the skin, and ideally to provide a persistent antiviral activity on the skin, or a hard surface, in order to control viral infections.

For example, rhinoviruses, influenza viruses, and adenoviruses are known to cause respiratory infections. Rhinoviruses are members of the picornavirus family, which is a family of "narked viruses" that lack an outer envelope. The human rhinoviruses are so termed because of their special adaptation to the nasopharyngeal region, and are the most important etiological agents of the common cold in adults and children. Officially there are 102 rhinovirus serotypes. Most of the picornaviruses isolated from the human respiratory system are acid labile, and this lability has become a defining characteristic of rhinoviruses.

Rhinovirus infections are spread from person to person by direct contact with virus-contaminated respiratory secretions. Typically, this contact is in the form of physical contact with a contaminated surface, rather than via inhalation of airborne viral particles.

Rhinovirus can survive on environmental surfaces for hours after initial contamination, and infection is readily transmitted by finger-to-finger contact, and by contaminated environmental surface-to-finger contact, if the newly contaminated finger then is used to rub an eye or touch the nasal mucosa. Therefore, virus contamination of skin and environmental surfaces should be minimized to reduce the risk of transmitting the infection to the general population.

Several gastrointestinal infections also are caused by viruses, particularly rotaviruses. For example, Norwalk virus causes nausea, vomiting (sometimes accompanied by diarrhea), and stomach cramps. This infection typically is spread from person to person by direct contact. Acute hepatitis A viral infection similarly can be spread by direct contact between one infected person and a nonimmune individual by hand-to-hand, hand-to-mouth, or aerosol droplet transfer, or by indirect contact when an uninfected individual comes into contact with a hepatitis A virus-contaminated solid object. Numerous other viral infections are spread similarly. The risk of transmitting such viral infections can be reduced significantly by inactivating or removing viruses from the hands and other environmental surfaces.

Common household phenol/alcohol disinfectants are effective in disinfecting contaminated environmental surfaces, but lack persistent virucidal activity. Hand washing is highly effective in disinfecting contaminated fingers, but again suffers from a lack of persistent activity. These shortcomings illustrate the need for improved virucidal compositions having a persistent activity against viruses, such as rhinoviruses and rotaviruses.

Antimicrobial personal care compositions are known in the art. In particular, antibacterial cleansing compositions, which typically are used to cleanse the skin and to destroy bacteria present on the skin, especially the hands, arms, and face of the user, are well-known commercial products.

Antibacterial compositions are used, for example, in the health care industry, food service industry, meat processing industry, and in the private sector by individual consumers. The widespread use of antibacterial compositions indicates the importance consumers place on controlling bacteria populations on skin. The paradigm for antibacterial compositions is to provide a substantial and broad spectrum reduction in bacterial populations quickly and without adverse side effects associated with toxicity and skin irritation. Such antibacterial compositions are disclosed in U.S. Patent Nos. 6,107,261 and 6,136,771.

One class of antibacterial personal care compositions is the hand sanitizer gels. This class of compositions is used primarily by medical personnel to disinfect the hands and fingers. A hand sanitizer gel is applied to, and rubbed into, the hands and fingers, and the composition is allowed to evaporate from the skin.

Hand sanitizer gels contain a high percentage of an alcohol, like ethanol. At the high percent of alcohol present in the gel, the alcohol itself acts as a disinfectant. In addition, the alcohol quickly evaporates to obviate wiping or rinsing skin treated with the sanitizer gel. Hand sanitizer gels containing a high percentage of an alcohol, i.e., about 40% or greater by weight of the composition, do not provide a persistent bacterial kill.

antibacterial cleansing compositions typically contain an active antibacterial agent, a surfactant, and various other ingredients, for example, dyes, fragrances, pH adjusters, thickeners, skin conditioners, and the like, in an aqueous and/or alcoholic carrier. Several different classes of antibacterial agents have been used in antibacterial cleansing compositions. Examples of antibacterial agents include a bisguanidine (e.g., chlorhexidine digluconate), diphenyl compounds, benzyl alcohols, trihalocarbanilides, quaternary ammonium compounds, ethoxylated phenols, and phenolic compounds, such as halo-substituted phenolic compounds, like PCMX (i.e., p-chloro-m-xylenol) and triclosan (i.e., 2,4,4'-trichloro-2'-hydroxydiphenylether). Antimicrobial compositions based on such antibacterial agents exhibit a wide range of antibacterial activity, ranging from low to high, depending on the microorganism to be controlled and the particular antibacterial composition. Most commercial antibacterial compositions generally offer a low to moderate antibacterial activity, and no reported antiviral activity.

Antimicrobial activity is assessed as the log reduction, or alternatively the percent reduction, in microbial populations provided by the antimicrobial composition. A 1-3 log reduction is preferred, a log reduction of 3-5 is most preferred, whereas a log reduction of less than 1 is least preferred, for a particular contact time, generally ranging from 15 seconds to 5 minutes. Thus, a highly preferred antimicrobial composition exhibits a 3-5 log reduction against a broad spectrum of microorganisms in a short contact time.

Virus control poses a more difficult problem than bacterial control. By sufficiently reducing bacterial populations, the risk of bacterial infection is reduced to acceptable levels. Therefore, a rapid antibacterial kill is desired. With respect to viruses, however, not only is a rapid kill desired, but a persistent antiviral activity also is required. This difference is because merely reducing a virus population is insufficient to reduce infection. In theory, a single virus can cause infection. Therefore, an essentially total, and persistent, antiviral activity is required, or at least desired, for an effective antiviral cleansing composition.

U.S. Patent No. 6,110,908 discloses a topical antiseptic containing a $C_{2-3}$ alcohol, a free fatty acid, and zinc pyrithione.

U.S. Patent No. 5,776,430 discloses a topical antimicrobial cleaner containing chlorhexidine and an alcohol. The compositions contain about 50% to 60%, by weight, denatured alcohol and about 0.65% to 0.85%, by weight, chlorhexidine. The composition is applied to the skin, scrubbed into the skin, then rinsed from the skin.

European Patent Application 0 604 848 discloses a gel-type hand disinfectant containing an antimicrobial agent, 40% to 90% by weight of an alcohol, and a polymer and a thickening agent in a combined weight of not more than 3% by weight. The gel is rubbed into the hands and allowed to evaporate to provide disinfected hands. The disclosed compositions often do not provide immediate sanitization and do not provide persistent antimicrobial efficacy.

In general, hand sanitizer gels typically contain: (a) at least 60% by weight ethanol or a combination of lower alcohols, such as ethanol and isopropanol, (b) water, (c) a gelling polymer, such as a crosslinked polyacrylate material, and (d) other ingredients, such as skin conditioners, fragrances, and the like. Hand sanitizer gels are used by consumers to effectively sanitize the hands, without, or after, washing with soap and water, by rubbing the hand sanitizer gel on the surface of the hands. Current commercial hand sanitizer gels rely on high levels of alcohol for disinfection and evaporation, and thus suffer from disadvantages. Specifically, because of the volatility of ethanol, the primary antimicrobial agent does not remain on the skin after use, thus failing to provide a persistent antimicrobial effect.

At alcohol concentrations below 60%, ethanol is not.recognized as an antiseptic. Thus, in compositions containing less than 60% alcohol, an additional antimicrobial compound is present to provide antimicrobial activity. Prior disclosures, however, have not addressed the issue of which composition ingredient in such an antimicrobial composition provides microbe control. Therefore, for formulations containing a reduced alcohol concentration, the selection of an antimicrobial agent that provides both a rapid antimicrobial effect and a persistent antimicrobial benefit is difficult.

U.S. Patent Nos. 6,107,261 and 6,136,771 disclose highly effective antibacterial compositions containing a phenolic antimicrobial agent. These patents disclose compositions that solve the problem of controlling bacteria on skin and hard surfaces, but are silent with respect to controlling viruses.

U.S. Patent Nos.-5,968,539; 6,106,851; and 6,113,933 disclose antibacterial compositions having a pH of about 3 to about 6. The compositions contain an antibacterial agent, an anionic surfactant, and a proton donor.

Antiviral compositions disclosed as inactivating or destroying pathogenic viruses, including rhinovirus, rotavirus, influenza virus, parainfluenza virus, respiratory syncytial virus, and Norwalk virus, also are known. For example, U.S. Patent No. 4,767,788 discloses the use of glutaric acid to inactivate or destroy viruses, including rhinovirus. U.S. Patent No. 4,975,217 discloses compositions containing an organic acid and an anionic surfactant, for formulation as a soap or lotion, to control viruses. U.S. Patent Publication 2002/0098159 discloses the use of a proton donating agent and a surfactant, including an antibacterial surfactant, to effect antiviral and antibacterial properties.

U.S. Patent No. 6,034,133 discloses a virucidal hand lotion containing malic acid, citric acid, and a $C_{1-6}$ alcohol. U.S. Patent No. 6,294,186 discloses combinations of a benzoic acid analog, such as salicyclic acid, and selected metal salts as being effective against viruses, including rhinovirus. U.S. Patent No. 6,436,885 discloses a combination of known antibacterial agents with 2-pyrrolidone-5-carboxylic acid, at a pH of 2 to 5.5, to provide antibacterial and antiviral properties.

Organic acids in personal washing compositions also have been disclosed. For example, WO 97/46218 and WO 96/06152 disclose the use of organic acids or salts, hydrotropes, triclosan, and hydric solvents in a surfactant base for antimicrobial cleansing compositions. These publications are silent with respect to antiviral properties.

Hayden et al., Antimicrobial Agents and Chemotherapy, 26:928-929 (1984), discloses interrupting the hand-to-hand transmission of rhinovirus colds through the use of a hand lotion having residual virucidal activity. The hand lotions, containing 2% glutaric acid, were more effective than a placebo in inactivating certain types of rhinovirus. However, the publication discloses that the glutaric acid-containing lotions were not effective against a wide spectrum of rhinovirus serotypes.

A virucidal tissue designed for use by persons infected with the common cold, and including citric acid, malic acid, and sodium lauryl sulfate, is known. Hayden et al., Journal of Infectious Diseases, 152:493-497 (1985), however, reported that use of paper tissues, either treated with virus-killing substances or untreated, can interrupt the hand-to-hand transmission of viruses. Hence, no distinct advantage in preventing the spread of rhinovirus colds can be attributed to the compositions incorporated into the virucidal tissues.

An efficacious antimicrobial composition effective against both bacteria and viruses has been difficult to achieve because of the fundamental differences between a bacteria and a virus. Although a number of antimicrobial cleansing products currently exist, taking a variety of product forms (e.g., deodorant soaps, hard surface cleaners, and surgical disinfectants), such antimicrobial products typically incorporate antimicrobial agents, e.g., a phenolic compound, and/or, harsh surfactants, which can dry out and irritate skin tissues. Ideally, personal cleansing products gently cleanse the skin, cause little or no irritation, and do not leave the skin overly dry after frequent use.

Accordingly, a need exists for an antimicrobial composition that is highly efficacious against a broad spectrum of microbes, including viruses and Gram positive and Gram negative bacteria, in a short time period, and wherein the composition can provide a persistent and broad spectrum antiviral activity, and is mild to the skin. Personal care products demonstrating improved mildness and a heightened level of viral and bacterial reduction are provided by the antimicrobial compositions of the present invention.

## SUMMARY OF THE INVENTION

The present invention is directed to a method of reducing and/or inactivating bacteria and a virus population on a hard, inanimate surface comprising contacting the surface with a composition for 30 seconds to achieve a log reduction of at least 2 against S. aureus, a log reduction of at least 2.5 against E. coli, and a log reduction of at least 4 against a nonevenloped virus, said composition comprising:

(a) 25 to 75 % by weight of a disinfecting alcohol, comprising one or more $C_{1-6}$ alcohol;
(b) 0.05 to 6 % by weight of an organic acid, comprising

(i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
(ii) a polymeric acid containing a plurality of carboxyl groups; and

(c) water,

wherein the composition has a pH of 5 or less at 25°C
and optionally rinsing the composition from the surface.

Regardless of the log P of the organic acid, a present antimicrobial composition provides a rapid and persistent control of nonenveloped viruses, and a fast broad spectrum bacteria kill. However, in one embodiment, the organic acid has a water-octanol partition coefficient, expressed as log P, of less than one, and the composition exhibits a synergistic activity against nonenveloped viruses. In another embodiment, the organic acid has a log P of one or greater, and the composition exhibits a synergistic activity agent bacteria. In yet another embodiment, the organic acid comprises a first organic acid having a log P less than one and an organic acid having a log P of one or greater, and the composition exhibits a synergistic activity against both nonenveloped viruses and bacteria.

Accordingly, one aspect of the present invention is to provide an antimicrobial composition that is highly effective at killing a broad spectrum of bacteria, including Gram positive and Gram negative bacteria such as *S. aureus, Salmonella choleraesuis, E. coli,* and *K. pneumoniae*, while simultaneously inactivating or destroying viruses harmful to human health, particularly nonenveloped viruses, like acid-labile viruses, and especially rhinoviruses, other acid-labile picornaviruses, and rotaviruses.

Another aspect of the present invention is to provide a liquid, antimicrobial composition comprising:

an antimicrobial composition, comprising:

(a) 25 to 75 % by weight of a disinfecting alcohol, comprising one or more $C_{1-8}$ alcohol;
(b) 0.05 to 6 % by weight of an organic acid, comprising

(i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
(ii) a polymeric acid containing a plurality of carboxyl groups; and

(c) water,
(d) optionally 0.01 to 5 % by weight of a gelling agent

wherein the composition has a pH of 5 or less at 25°C.

These and other novel aspects and advantages of the present invention are set forth in the following, nonlimiting detailed description of the preferred embodiments.

## DETAILED DESCRIPTION OF THE PREFERRED EMBODIMENTS

Personal care products incorporating an active antimicrobial agent have been known for many years. Since the introduction of antimicrobial personal care products, many claims have been made that such products provide antimicrobial properties. To be most effective, an antimicrobial composition should provide a high log reduction against a broad spectrum of organisms in as short a contact time as possible. Ideally, the composition also should inactivate viruses.

As presently formulated, most commercial liquid antibacterial soap compositions provide a poor to marginal time kill efficacy; i.e., rate of killing bacteria. These compositions do not effectively control viruses.

Antimicrobial hand sanitizer compositions typically do not contain a surfactant and rely upon a high concentration of an alcohol to control bacteria. The alcohols evaporate and, therefore, cannot provide a persistent bacterial control.

The alcohols also can dry and irritate the skin.

Most current products especially lack efficacy against Gram negative bacteria, such as *E. coli*, which are of particular concern to human health. Compositions do exist, however, that have an exceptionally high broad spectrum antibacterial efficacy, as measured by a rapid kill of bacteria (i.e., time kill), which is to be distinguished from persistent kill. These products also lack a sufficient antiviral activity.

The present antimicrobial compositions provide excellent broad spectrum antiviral and antibacterial efficacy and significantly improve antiviral efficacy compared to prior compositions that incorporate a high percentage of an alcohol, i.e., 40% or greater, by weight. The basis of this improved efficacy is (a) the discovery that a combination of a disinfecting alcohol and an organic acid, and especially an organic acid having a log P of less than about 1, substantially improves antiviral efficacy, and (b) the pH of a surface after application of the composition to the surface.

A disinfecting alcohol and an organic acid having a log P of less than one act synergistically to control nonenveloped viruses. A disinfecting alcohol and an organic acid having a log P of one or greater act synergistically to substantially improve antibacterial efficacy. A combination of a first organic acid having a log P less than one and a second organic acid having a log P of one or greater, with a disinfecting alcohol, provides a synergistic improvement in the control of nonenveloped viruses and Gram positive and Gram negative bacteria.

Although compositions containing an antimicrobial agent, like triclosan, have demonstrated a rapid and effective antibacterial activity against Gram positive and Gram negative bacteria, control of viruses has been inadequate. Virus control on skin and inanimate surfaces is very important in controlling the transmission of numerous diseases.

For example, rhinoviruses are the most significant microorganisms associated with the acute respiratory illness referred to as the "common cold." Other viruses, such as parainfluenza viruses, respiratory syncytial viruses (RSV), enteroviruses, and coronaviruses, also are known to cause symptoms of the "common cold," but rhinoviruses are theorized to cause the greatest number of common colds. Rhinoviruses also are among the most difficult of the cold-causing viruses to control, and have an ability to survive on a hard dry surface for more than four days. In addition, most viruses are inactivated upon exposure to a 70% ethanol solution. However, rhinoviruses remain viable upon exposure to ethanol.

Because rhinoviruses are the major known cause of the common cold, it is important that a composition having antiviral activity is active against the rhinovirus. Although the molecular biology of rhinoviruses is now understood, finding effective methods for preventing colds caused by rhinoviruses, and for preventing the spread of the virus to noninfected subjects, has been fruitless.

It is known that iodine is an effective antiviral agent, and provides a persistent antirhinoviral activity on skin. In experimentally induced and natural cold transmission studies, subjects who used iodine products had significantly fewer colds than placebo users. This indicates that iodine is effective for prolonged periods at blocking the transmission of rhinoviral infections. Thus, the development of products that deliver both immediate and persistent antiviral activity would be effective in reducing the incidence of colds. Likewise, a topically applied composition that exhibits antiviral activity would be effective in preventing and/or treating diseases caused by other nonenveloped viruses, including acid-labile viruses.

A rotavirus also is a nonenveloped, double-shelled virus that is stable in the environment. Rotavirus infection is an infection of the digestive tract, and is the most common cause of severe diarrhea among children, resulting in over 50,000 hospitalizations yearly in the U.S. alone. Rotaviral infections are particularly problematic in close communities, such as child care facilities, geriatric facilities, family homes, and children's hospitals.

The most common mode of transmitting rotavirus is person to person spread through contaminated hands, but transmission also can occur through ingestion of contaminated water or food, or through contact with contaminated surfaces. The rotavirus then enters the body through contact with the mouth.

It is known that washing hands and hard surfaces with soap and/or other cleansers does not kill rotavirus, but helps prevent its spread. An oral rotavirus vaccine has been approved for use in children in the U.S., but its use is not recommended because of a severe adverse side effect. Because no other effective way to eliminate rotavirus, or its spread, is currently available, workers in close communities, especially those catering to children, must adhere to strict hygienic practices to help curtail the spread of rotavirus. An improved composition having enhanced antiviral efficacy, including persistent antiviral efficacy, in inactivating rotaviruses would further curtail the spread of rotavirus infections.

virucidal means capable of inactivating or destroying a virus. As used herein, the term "persistent antiviral efficacy" or "persistent antiviral activity" means leaving a residue or imparting a condition on animate (e.g., skin) or inanimate surfaces that provides significant antiviral activity for an extended time after application. A composition of the present invention provides a persistent antiviral efficacy, i.e., preferably a log reduction of at least 3, and more preferably a log reduction of at least log 4, against nonenveloped viruses, including acid-labile viruses, such as rhinovirus and rotavirus serotypes, within 30 seconds of contact with the composition. Antiviral activity is maintained for at least about 0.5 hour, preferably at least about one hour, and more preferably for at least about two hours, at least about three hours, or at least about four hours after contact with the composition. In some preferred embodiments, antiviral activity is maintained for about six to about eight hours after contact with the composition. The methodology utilized to determine a persistent antiviral efficacy is discussed below.

The antimicrobial compositions of the present invention are highly effective in providing a rapid and broad spectrum control of bacteria, and a rapid and persistent control of nonenveloped viruses. The highly effective compositions comprise a disinfecting alcohol and a virucidally effective amount of an organic acid.

The disinfecting alcohol and an organic acid having a log P of less than about 1 act synergistically to control a broad spectrum of nonenveloped viruses. The disinfecting alcohol and an organic acid having a log P of 1 or greater act synergistically to control a broad spectrum of bacteria. A composition containing a first organic acid having a log P of less than one and a second organic acid having a log P of one or greater act synergistically to control a broad spectrum of nonenveloped viruses and a broad spectrum of Gram positive and Gram negative bacteria.

The compositions are surprisingly mild to the skin, and noncorrosive to inanimate surfaces. Thus, mild and effective compositions that solve the problem of bacterial and viral control are provided to consumers.

The present compositions provide an effective and persistent inactivation of nonenveloped viruses. Nonenveloped viruses include, but are not limited to, adenoviruses, caulimoviruses, papovaviruses, phycodna viruses, circoviruses, parvoviruses, birnaviruses, rotoviruses (including rotavirus gastroenteritis), astroviruses, caliciviruses (including Norwalk virus), potyviruses, and picornaviruses (including rhinovirus, polio virus, and hepatitis A virus).

The antimicrobial compositions of the present invention are highly efficacious in household cleaning applications (e.g., hard surfaces, like floors, counter-tops, tubs, dishes, and soft cloth materials, like clothing), personal care applications (e.g., lotions, shower gels, soaps, shampoos, and wipes), and industrial and hospital applications (e.g., sterilization of instruments, medical devices, and gloves). The present compositions efficaciously and rapidly disinfect surfaces that are infected or contaminated with Gram negative bacteria, Gram positive bacteria, and nonenveloped viruses (e.g., rhinovirusee and rotaviruses). The present compositions also provide a persistent antiviral effectiveness.

The present compositions can be used in vitro and *in vivo. In vitro* means in or on nonliving things, especially on inanimate objects having hard or soft surfaces located or used where preventing viral transmission is desired, most especially on objects that are touched by human hands. In vivo means in or on animate objects, especially on mammal skin, and particularly on hands.

As illustrated in the following nonlimiting embodiments, an antimicrobial composition of the present invention comprises:

(a) 25 to 75 % by weight of a disinfecting alcohol, comprising one or more $C_{1-8}$ alcohol;
(b) 0.05 to 6 % by weight of an organic acid, comprising

(i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
(ii) a polymeric acid containing a plurality of carboxyl groups; and

(c) water,
(d) optionally 0.01 to 5 % by weight of a gelling agent

wherein the composition has a pH of 5 or less at 25°C. In preferred embodiments, the composition contains an optional gelling agent.

The compositions exhibit a log reduction against Gram positive bacteria of about 2 after 30 seconds contact. The compositions also exhibit a log reduction against Gram negative bacteria of about 2.5. after 30 seconds contact. The compositions further exhibit a log reduction against nonenveloped viruses, including acid-labile viruses, such as rhinovirus and rotavirus serotypes of about 5 after 30 seconds contact, and a log reduction against these acid-labile viruses of at least 3 about five hours after contact, and at least about 2 about six to about eight hours after contact. The compositions also are mild, and it is not necessary to rinse or wipe the compositions from the skin.

In accordance with the invention, a present antimicrobial composition can further comprise additional optional ingredients disclosed hereafter, like hydrotropes, polyhydric solvents, gelling agents, pH adjusters, vitamins, dyes, skin conditioners, and perfumes. The compositions are free of intentionally added cleansing surfactants, like anionic surfactants, and active antimicrobial agents, like phenolic and quaternary ammonium antimicrobial agents.

The following ingredients are present in an Antimicrobial composition of the present invention.

## A. Disinfecting Alcohol

Antimicrobial compositions of the present invention contain comprising one or more $C_{1-6}$ alcohol 25% to 75%, by weight, of a disinfecting alcohol. Preferred embodiments of the present invention contain about 30% to about 75%, by weight, of a disinfecting alcohol. Most preferred embodiments contain about 30% to about 70%, by weight, of a disinfecting alcohol.

As used herein, the term "disinfecting alcohol" is a water-soluble alcohol containing one to six carbon atoms. Disinfecting alcohols include, but are not limited to, methanol, ethanol, propanol, and isopropyl alcohol.

## B. Organic Acid

A present antimicrobial composition also contains 0.05 to 6 % by weight of an organic acid, comprising

(i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
(ii) a polymeric acid containing a plurality of carboxyl groups.

The organic acid acts synergistically with the disinfecting alcohol to provide a rapid control of nonenveloped viruses and/or bacteria, and provides a persistent viral control.

In particular, an organic acid is present in the composition in a sufficient amount such that the pH of the animate or inanimate surface contacted by the composition is lowered to degree wherein a persistent viral control is achieved. This persistent viral control is achieved regardless of whether the composition is rinsed from, or allowed to remain on, the contacted surface. The organic acid remains at least partially undissociated in the composition, and remains so when the composition is diluted, or during application and rinsing.

Upon application to a surface, such as human skin, the pH of the surface is sufficiently lowered such that a persistent viral control is achieved. In preferred embodiments, a residual amount of the organic acid remains on the skin, even after a rinsing step, in order to impart a persistent viral control. However, even if the organic acid is essentially completely rinsed from the surface, the surface pH has been sufficiently lowered to impart a viral control for at least 0.5 hour.

Preferably an organic acid is included in a present composition in an amount of 0.1% to 5%, by weight of the composition. To achieve the full advantage of the present invention, the organic acid is present in an amount of 0.15% to 4%, by weight of the composition. The amount of organic acid is related to the class of organic acid used, and to the identity of the specific acid or acids used.

An organic acid included in a present anti-microbial, composition preferably does not penetrate the surface to which it is applied, e.g., remains on the skin surface as opposed to penetrating the skin. The organic acid, therefore, preferably is a hydrophobic organic acid.

In one embodiment of the present invention, the organic acid has a log P of less than one, and preferably less than 0.75. To achieve the full advantage of the present invention, the organic acid has a log P of less than 0.5. In this embodiment, the disinfecting alcohol and organic acid act synergistically to provide an effective and persistent viral control.

In another embodiment, the organic acid has a log.P of 1 or greater, for example, 1 to about 100. In this embodiment, the disinfecting alcohol and organic acid affectively control nonenveloped viruses and also act synergistically to control a broad spectrum of bacteria.

It is envisioned that, by incorporating a first organic acid having a log P of less than one and a second organic acid having a log P of 1 or greater into a present composition, the first and second organic acids act synergistically with the disinfecting alcohol to provide a persistent control of nonenveloped viruses and a broad spectrum bacteria control.

As used herein, the term "log P" is defined as the log of the water-octanol partition coefficient, i.e., the log of the ratio $P_w/P_o$, wherein $P_w$ is the concentration of an organic acid in water and $P_o$ is the concentration of the organic acid in octanol, at equilibrium and 25°C. The water-octanol coefficient can be determined by the U.S. Environmental Protection Agency Procedure, "OPTS 830.7560 Partition Coefficient (n-botanol/Water), Generator Column Method" (1996).

Organic acids having a log P less than one typically are water insoluble, e.g., have a water solubility of less than about 0.5 wt% at 25°C.

The organic acid comprises more than one polycarboxylic acid selected from the group consisting of malonic acid, succinic acid, glutaric acid, adipic acid, terephthalic acid, phthalic acid, pimelic acid, suberic acid, azelaic acid, sebacic acid, fumaric acid, maleic acid, tartaric acid, malic acid, citric acid, maleic acid, aconitic acid, and mixtures thereof.

The organic acid comprises a polymeric carboxylic acid. The polymeric acid has a molecular weight of about 500 g/mol to 10,000,000 g/mol, and includes homopolymers, copolymers, and mixtures thereof. The polymeric acid preferably is capable of forming a substantive film on a surface and has a glass transition temperature, $T_g$, of less than 25°C, preferably less than 20°C, and more preferably less than about 15°C. The glass transition temperature is the temperature at which an amorphous material, such as a polymer, changes from a brittle, vitreous state to a plastic state. The $T_g$ of a polymer is readily determined by persons skilled in the art using standard techniques.

The polymeric acids are uncrosslinked or only very minimally crosslinked. The polymeric acids typically are prepared from ethylenically unsaturated monomers having at least one hydrophilic moiety, such as carboxyl, carboxylic acid anhydride; sulfonic acid, and sulfate. The polymeric acid can contain a comonomer, such as styrene or an alkene, to increase the hydrophobicity of the polymeric acid.

Examples of monomers used to prepare the polymeric organic acid include, but are not limited to:

(a) Carboxyl group-containing monomers, e.g., monoethylenically unsaturated mono- or polycarboxylic acids, such as acrylic acid, methacrylic acid, maleic acid, fumaric acid, crotonic acid, sorbic acid, itaconic acid, ethacrylic acid, α-chloroacrylic acid, α-cyano-acrylic acid, β-methlacrylic acid (crotonic acid), α-phenylacrylic acid, β-acryloxypropionic acid, sorbic acid, α-chlorosorbic acid, angelic acid, cinnamic acid, p-chlorocinnamic acid, β-stearylacrylic acid, citraconic acid, mesaconic acid, glutaconic acid, aconitic acid, tricarboxyethylene, and cinnamic acid;

(b) Carboxylic acid anhydride group-containing monomers, e.g., monoethylenically unsaturated polycarboxylic acid anhydrides, such as maleic anhydride; and

(c) Sulfonic acid group-containing monomers, e.g., aliphatic or aromatic vinyl sulfonic acids, such as vinylsulfonic acid, allylsulfonic acid, vinyltoluenesulfonic acid, styrenesulfonic acid, sulfoethyl (meth)acrylate, 2-acrylamido-2-methylpropane sulfonic acid, sulfopropyl (meth)acrylate, and 2-hydroxy-3-(meth)acryloxy propyl sulfonic acid.

The polymeric acid can contain other copolymerizable units, i.e., other monoethylenically unsaturated comonomers, well known in the art, as long as the polymer is substantially, i.e., at least 10%, and preferably at least 25%, acid group containing monomer units. To achieve the full advantage of the present invention, the polymeric acid contains at least 50%, and more preferably, at least 75%, and up to 100%, acid group containing monomer units. The other copolymerizable units, for example, can be styrene, an alkene, an alkyl acrylate, or an alkyl methacrylate. The polymeric acid also can be partially neutralized, which assists dispersion of the polymeric acid into a composition. However, a sufficient number of the acid groups remain unneutralized to reduce skin pH and impart a persistent antiviral activity.

One preferred polymeric acid is a polyacrylic acid, either a homopolymer or a copolymer, for example, a copolymer of acrylic acid and an alkyl acrylate and/or alkyl methacrylate. Another preferred polymeric acid is a homopolymer or a copolymer of methacrylic acid.

Exemplary polymeric acids useful in the present invention include, but are not limited to:

| | |
|---|---|
| Carbomers | (CARBOPOL 910, 934, 934P, 940, 941, ETD 2050; ULTREZ 10, 21) (CARBOPOL ETD 2050) |
| Acrylates/C20-30 Alkyl Acrylate Crosspolymer | (ULTREZ 20) |
| Acrylates/Beheneth 25 Methacrylate Copolymer | (ACULYN 28) |
| Acrylates/Steareth 20 Methacrylate Copolymer | (ACULYN 22) |
| Acrylates/Steareth 20 Methacrylate Crosspolymer | (ACULYN 88) (ACULYN 88) |
| Acrylates Copolymer | (CAPIGEL 98) |
| Acrylates Copolymer | (AVALURE AC) |
| Acrylates/Palmeth 25 Acrylate Copolymer | (SYNTHALEN 2000) |
| Ammonium Acrylate Copolymers | |
| Sodium Acrylate/vinyl Alcohol Copolymer | |
| Sodium Polymethacrylate | |
| Acrylamidopropyltrimonium Chloride/Acrylates Copolymer | |
| Acrylates/Acrylamide Copolymer | |
| Acrylates/Ammonium Methacrylate Copolymer | |
| Acrylates/C10-30 Alkyl Acrylate Crosspolymer | |
| Acrylates/Diacetoneacrylamide Copolymer | |
| Acrylates/Octylacrylamide Copolymer | |
| Acrylates/VA Copolymer | |
| Acrylic Acid/Acrylonitrogens Copolymer | |

In a preferred embodiment of the present invention, the organic acid comprises one or more polycarboxylic acid, e.g., citric acid, malic acid, tautaric acid, or a mixture of any two or all three of these acids, and a polymeric acid containing a plurality of carboxyl groups, for example, homopolymers and copolymers of acrylic acid or methacrylic acid.

**C. Carrier**

The carrier of the present antimicrobial composition comprises water.

**D. Optional ingredients**

An antimicrobial composition of the present invention also can contain optional ingredients well known to persons skilled in the art. The particular optional ingredients and amounts that can be present in the composition are discussed hereafter.

The optional ingredients are present in a sufficient amount to perform their intended function and not adversely affect the antimicrobial efficacy of the composition, and in particular not adversely affect the synergistic effect provided by the disinfecting alcohol and organic acid. Optional ingredients typically are present, individually or collectively, from 0% to about 50%, by weight of the composition.

Classes of optional ingredients include, but are not limited to, hydrotropes, polyhydric solvents, gelling agents, dyes, fragrances, pH adjusters, thickeners, viscosity modifiers, chelating agents, skin conditioners, emollients, preservatives, buffering agents, antioxidants, chelating agents, opacifiers, and similar classes of optional ingredients known to persons skilled in the art.

A hydrotrope, if present at all, is present in an amount of about 0.1% to about 30%, and preferably about 1% to about 20%, by weight of the composition. To achieve the full advantage of the present invention, a composition can contain about 2% to about 15%, by weight, of a hydrotrope.

A hydrotrope is a compound that has an ability to enhance the water solubility of other compounds. A hydrotrope utilized in the present invention lacks surfactant properties, and typically is a short-chain alkyl aryl sulfonate. Specific examples of hydrotropes include, but are not limited to, sodium cumene sulfonate, ammonium cumene sulfonate, ammonium xylene sulfonate, potassium toluene sulfonate, sodium toluene sulfonate, sodium xylene sulfonate, toluene sulfonic acid, and xylene sulfonic acid. Other useful hydrotropes include sodium polynaphthalene sulfonate, sodium polystyrene sulfonate, sodium methyl naphthalene sulfonate, sodium camphor sulfonate, and disodium succinate.

A polyhydric solvent, if present at all, is present in an amount of about 0.1% to about 30%, and preferably about 5% to about 30%, by weight of the composition. To achieve the full advantage of the present invention, the polyhydric solvent is present in an amount of about 10% to about 30% by weight of the composition. In contrast to a disinfecting alcohol, a polyhydric solvent contributes minimally, if at all, to the antimicrobial efficacy of the present composition.

The term "polyhydric solvent" as used herein is a water-soluble organic compound containing two to six, and typically two or three, hydroxyl groups. The term "water-soluble." means that the polyhydric solvent has a water solubility of at least 0.1 g of polyhydric solvent per 100 g of water at 25°C. There is no upper limit to the water solubility of the polyhydric solvent, e.g., the polyhydric solvent and water can be soluble in all proportions.

The term polyhydric solvent, therefore, encompasses water-soluble diols, triols, and polyols. Specific examples of hydric solvents include, but are not limited to, ethylene glycol, propylene glycol, glycerol, diethylene glycol, dipropylene glycol, tripropylene glycol, hexylene glycol, butylene glycol, 1,2,6-hexanetriol, sorbitol, PEG-4, and similar polyhydroxy compounds.

Other specific classes of optional ingredients include inorganic phosphates, sulfates, and carbonates as buffering agents; EDTA and phosphates as chelating agents; and acids and bases as pH adjusters.

Examples of preferred classes of optional basic pH adjusters are ammonia; mono-, di-, and tri-alkyl amines; mono-, di-, and tri-alkanolamines; alkali metal and alkaline earth metal hydroxides; and mixtures thereof. However, the identity of the basic pH adjuster is not limited, and any basic pH adjuster known in the art can be used. Specific, nonlimiting examples of basic pH adjusters are ammonia; sodium, potassium, and lithium hydroxide; monoethanolamine; triethylamine; isopropanolamine; diethanolamine; and triethanolamine.

Examples of preferred classes of optional acidic pH adjusters are the mineral acids. Nonlimiting examples of mineral acids are hydrochloric acid, nitric acid, phosphoric acid, and sulfuric acid. The identity of the acidic pH adjuster is not limited and any acidic pH adjuster known in the art, alone or in combination, can be used.

An optional alkanolamide to provide composition thickening can be, but is not limited to, cocamide MEA, cocamide DEA, soyamide DEA, lauramide DEA, oleamide MIPA, stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA, and mixtures thereof. Alkanolamides are noncleansing surfactants and are added, if at all, in small amounts to thicken the composition.

The present antimicrobial compositions also can contain about 0.01% to about 5%, by weight, and preferably 0.10% to about 3%, by weight, of an optional gelling agent. To achieve the full advantage of the present invention, the antimicrobial compositions contain about 0.25% to about 2.5%, by weight, of a gelling agent. The antimicrobial compositions typically contain a sufficient amount of gelling agent such that the composition is a viscous liquid, gel, or semisolid that can be easily applied to, and rubbed on, the skin or other surface. Persons skilled in the art are aware of the type and amount of gelling agent to include in the composition to provide the desired composition viscosity or consistency.

The term "gelling agent" as used here and hereafter refers to a compound capable of increasing the viscosity of a water-based composition, or capable of converting a water-based composition to a gel or semi-solid. The gelling agent, therefore, can be organic in nature, for example, a natural gum or a synthetic polymer, or can be inorganic in nature.

As previously stated, the present compositions are free of a cleansing surfactant and an antimicrobial agent. A cleansing surfactant and antimicrobial agent are not intentionally added to a present antimicrobial composition, but may be present in an amount of 0% to about 0.5%, by weight, because a surfactant may be present in a commercial form of a gelling agent to help disperse the gelling agent in water. A surfactant also may be present as an additive or by-product in other composition ingredients. An antimicrobial agent may be present in a composition ingredient as a preservative.

The following are nonlimiting examples of gelling agents that can be used in the present invention. In particular, the following compounds, both organic and inorganic, act primarily by thickening or gelling the aqueous portion of the composition:

acacia, agar, algin, alginic acid, ammonium alginate, ammonium chloride, ammonium sulfate, amylopectin, attapulgite, bentonite, $C_{9-15}$ alcohols, calcium acetate, calcium alginate, calcium carrageenan, calcium chloride, caprylic alcohol, carboxymethyl hydroxyethylcellulose, carboxymethyl hydroxypropyl guar, carrageenan, cellulose, cellulose gum, cetearyl alcohol, cetyl alcohol, corn starch, damar, dextrin, dibenzylidine sorbitol, ethylene dihydrogenated tallowamide, ethylene dioleamide, ethylene distearamide, gelatin, guar gum, guar hydroxypropyltrimonium chloride, hectorite, hyaluronic acid, hydrated silica, hydroxybutyl methylcellulose, hydroxyethylcellulose, hydroxyethyl.ethylcellulose, hydroxyethyl stearamide-MIPA, hydroxypropylcellulose, hydroxypropyl guar, hydroxypropyl methylcellulose, isocetyl alcohol, isostearyl alcohol, karaya gum, kelp, lauryl alcohol, locust bean gum, magnesium aluminum silicate, magnesium silicate, magnesium trisilicate, methoxy PEG-22/dodecyl glycol copolymer, methylcellulose, microcrystallinc cellulose, montmorillonite, myristyl alcohol, oat flour, oleyl alcohol, palm kernel alcohol, pectin, PEG-2M, PEG-5M, polyvinyl alcohol, potassium alginate, potassium carrageenan, potassium chloride, potassium sulfate, potato starch, propylene glycol alginate, sodium carboxymethyl dextran, sodium carrageenan, sodium cellulose sulfate, sodium chloride, sodium silicoaluminate, sodium sulfate, stearalkonium bentonite, stearalkonium hectorite, stearyl alcohol, tallow alcohol, TEA-hydrochloride, tragacanth gum, tridecyl alcohol, tromethamine magnesium aluminum silicate, wheat flour, wheat starch, xanthan gum, and mixtures thereof.

The following additional nonlimiting examples of gelling agents act primarily by thickening the nonaqueous portion of the composition:

abietyl alcohol, acrylinoleic acid, aluminum behenate, aluminum caprylate, aluminum dilinoleate, aluminum distearate, aluminum isostearates/laurates/ palmitates or stearates, aluminum isostearates/ myristates, aluminum isostearates/palmitates, aluminum isostearates/stearates, aluminum lanolate, aluminum myristates/palmitates, aluminum stearate, aluminum stearates, aluminum tristearate, beeswax, behenamide, behenyl alcohol, butadiene/acrylonitrile copolymer, a $C_{29-70}$ acid, calcium behenate, calcium stearate, candelilla wax, carnauba, ceresin, cholesterol, cholesteryl hydroxy-stearate, coconut alcohol, copal, diglyceryl stearate malate, dihydroabietyl alcohol, dimethyl lauramine oleate, dodecanedioic acid/cetearyl alcohol/glycol copolymer, erucamide, ethylcellulose, glyceryl triacetyl hydroxystearate, glyceryl triacetyl ricinoleate, glycol dibehenate, glycol dioctanoate, glycol distearate, hexanediol distearate, hydrogenated $C_{6-14}$ olefin polymers, hydrogenated castor oil, hydrogenated cottonseed oil, hydrogenated lard, hydrogenated menhaden oil, hydrogenated palm kernel glycerides, hydrogenated palm kernel oil, hydrogenated palm oil, hydrogenated polyisobutene, hydrogenated soybean oil, hydrogenated tallow amide, hydrogenated tallow glyceride, hydrogenated vegetable glyceride, hydrogenated vegetable glycerides, hydrogenated vegetable oil, hydroxypropylcellulose, isobutylene/isoprene copolymer, isocetyl stearoyl stearate, Japan wax, jojoba wax, lanolin alcohol, lauramide, methyl de-hydroabietate, methyl hydrogenated rosinate, methyl rosinate, methylstyrene/vinyltoluene copolymer, microcrystalline wax, montan acid wax, montan wax, myristyleicosanol, myristyloctadecanol, octadecene/maleic anhydride copolymer, octyldodecyl stearoyl stearate, oleamide, oleostearine, ouricury wax, oxidized polyethylene, ozokerite, palm kernel alcohol, paraffin, pentaerythrityl hydrogenated rosinate, pentaerythrityl rosinate, pentaerythrityl tetraabietate, pentaerythrityl tetrabehenate, pentaerythrityl tetraoctanoate, pentaerythrityl tetraoleate, pentaerythrityl tetrastearate, phthalic anhydride/glycerin/glycidyl decanoate copolymer, phthalic/trimellitic/glycols copolymer, polybutene, polybutylene terephthalate, polydipentene, polyethylene, polyisobutene, polyisoprene, polyvinyl butyral, polyvinyl laurate, propylene glycol dicaprylate, propylene glycol dicocoate, propylene glycol diisononanoate, propylene glycol dilaurate, propylene glycol dipelargonate, propylene glycol distearate, propylene glycol diundecanoate, PVP/eicosene copolymer, PVP/hexadecene, copolymer, rice bran wax, stearalkonium bentonite, stearalkonium hectorite, stearamide, stearamide DEA-distearate, stearamide DIBA-stearate, stearamide MEA-stearate, stearone, stearyl alcohol, stearyl erucamide, stearyl stearate, stearyl stearoyl stearate, synthetic beeswax, synthetic wax, trihydroxystearin, triisononanoin, triisostearin, triisostearyl trilinoleate, trilaurin, trilinoleic acid, trilinolein, trimyristin, triolein, tripalmitin, tristearin, zinc laurate, zinc myristate, zinc neodecanoate, zinc rosinate, zinc stearate, and mixtures thereof.

Exemplary gelling agents useful in the present invention include, but are not limited to,

| | |
|---|---|
| Polyethylene Glycol & Propylene Glycol & Water | (ACULYN 44) |
| Ammonium Acrylatedimethyltaurate/VP Copolymer | (ARISTOFLEX AVC) |
| Glyceryl Stearate & PEG 100 Stearate | (ARLACEL 165) |
| Polyethylene (2) Stearyl Ether | (BRIJ 72) |
| Polyoxyethylene(21)Stearyl Ether | (BRIJ 721) |
| Silica | (CAB-O-SIL) |
| Polyquaternium 10 | (CELQUAT CS230M) |
| Cetyl Alcohol | |
| Cetearyl Alcohol & Cetereth 20 | (COSMOWAX P) |
| Cetearyl Alcohol & Dicetyl Phosphate & Ceteth-10 Phosphate | (CRODAFOS CES) |
| Ceteth-20 Phosphate & Cetearyl Alcohol & Dicetyl Phosphate | (CRODAFOS CS-20 Acid) |
| Cetearyl Alcohol & Cetereth 20 | (EMULGADE NI 1000) |
| Sodium Magnesium Silicate, | (LAPONITE XLG) |
| Cetyl Alcohol & Stearyl Alcohol & Stearalkonium Chloride & Dimethyl Stearamine & Lactic Acid | (MACKADET CBC) |
| Cetearyl Alcohol & Stearamidopropyldimethylamine & Stearamidopropylalkonium Chloride | (MACKERNIUM Essential) |
| Stearalkonium Chloride | (MACKERNIUM SDC-85) |
| Cetearyl Alcohol & Stearamidopropyldimethylamine & Stearamidopropylalkonium Chloride & Silicone Quaternium 16 | (MACKERNIUM Ultra) |
| Cetearyl Alcohol & Cetearyl Glucoside | (MONTANOV 68EC) |
| Hydroxyethylcellulose | (NATROSOL 250 HHR CS) |
| Polyquaternium-37 & Mineral Oil & Trideceth-6 | (SALCARE SC 95) |
| Polyquaternium-32 & Mineral Oil & Trideceth-6 | (SALCARE SC 96) |
| Stearic Acid | |
| Cetyl Hydroxyethylcellulose | (NATROSOL Plus 330 CS) |
| Polyvinyl Alcohol, PVP-K30, Propylene Glycol | |
| Stearic Acid, Behenyl Alcohol, Glycerol Stearate, Lecithin, C12-16 Alcohols, Palmic Acid | (PROLIPID 141) |
| Beeswax | (saponified beeswax) |
| Beeswax | (synthetic beeswax) |
| Water, Beeswax, Sesame Oil, Lecithin, Methyl paraben | (beesmilk) |
| Polyquaternium 10 | (CELQUAT SC240C) |

(continued)

| Sodium Acrylate/Sodium Acrylodimethyl Taurate Copolymer & Isohexadecane & Polysorbate 80 | (SIMULGEL EG) |
| Polyquaternium 44 | (LUVIQUAT Care) |

### E. pH

The pH of a present antimicrobial composition is less than about 5, and preferably less than about 4.5 at 25°C. To achieve the full advantage of the present invention, the pH is less than about 4. Typically, the pH of a present composition is about 2 to less than about 5, and preferably about 2.5 to about 4.5.

The pH of the composition is sufficiently low such that at least a portion of the organic acid is in the protonated form. The organic acid then has the capability of lowering surface pH, such as skin pH, to provide an effective viral control, without irritating the skin. The organic acid also deposits on the skin, and resists removal by rinsing, to provide a persistent antiviral effect.

To demonstrate the new and unexpected results provided by the antimicrobial compositions of the present invention, the following examples are prepared, and the ability of the compositions to control Gram positive and Gram negative bacteria, and to control rhinovirus, is determined. The weight percentage listed in each of the following examples represents the actual, or active, weight amount of each ingredient present in the composition. The compositions are prepared by blending the ingredients, as understood by those skilled in the art and as described below.

The following methods are used in the preparation and testing of the examples:

a) Determination of Rapid Germicidal (Time Kill). Activity of Antibacterial Products. The activity of antibacterial compositions is measured by the time kill method, whereby the survival of challenged organisms exposed to an antibacterial test composition is determined as a function of time. In this test, a diluted aliquot of the composition is brought into contact with a known population of test bacteria for a specified time period at a specified temperature. The test composition is neutralized at the end of the time period, which arrests the antibacterial activity of the composition. The percent or, alternatively, log reduction from the original bacteria population is calculated.

In general, the time kill method is known to those skilled in the art.

The composition can be tested at any concentration up to 100%. The choice of which concentration to use is at the discretion of the investigator, and suitable concentrations are readily determined by those skilled in the art. For example, viscous samples usually are tested at 50% dilution, whereas nonviscous samples are not diluted. The test sample is placed in a sterile 250 ml beaker equipped with a magnetic stirring bar and the sample volume is brought to 100 ml, if needed, with sterile deionized water. All testing is performed in triplicate, the results are combined, and the average log reduction is reported.

The choice of contact time period also is at the discretion of the investigator. Any contact time period can be chosen. Typical contact times range from 15 seconds to 5 minutes, with 30 seconds and 1 minute being typical contact times. The contact temperature also can be any temperature, typically room temperature, or about 25 degrees Celsius.

The bacterial suspension, or test inoculum, is prepared by growing a bacterial culture on any appropriate solid media (e.g., agar). The bacterial population then is washed from the agar with sterile physiological saline and the population of the bacterial suspension is adjusted to about $10^8$ colony forming units per ml (cfu/ml).

The table below lists the test bacterial cultures used in the tests and includes the name of the bacteria, the ATCC (American Type Culture Collection) identification number, and the abbreviation for the name of the organism used hereafter. *S. aureus* is a Gram positive bacteria, whereas *E. coli, K. pneum,* and *S. choler.* are Gram negative bacteria.

| Organism Name | ATCC # | Abbreviation |
|---|---|---|
| *Staphylococcus aureus* | 6538 | *S. aureus* |
| *Escherichia coli* | 11229 | *E. coli* |
| *Klebsiella pneumoniae* | 10031 | *K. pneum.* |
| *Salmonella choleraesuis* | 10708 | *S. choler.* |

The beaker containing the test composition is placed in a water bath (if constant temperature is desired), or placed on a magnetic stirrer (if ambient laboratory temperature is desired). The sample then is inoculated with 1.0 ml of the test bacteria suspension. The inoculum is stirred with the test composition for the predetermined contact time. When the contact time expires, 1.0 ml of the test composition/bacteria mixture is transferred into 9.0 ml of Neutralizer Solution.

13

Decimal dilutions to a countable range then are made. The dilutions can differ for different organisms. Selected dilutions are plated in triplicate on TSA+ plates (TSA+ is Trypticase Soy Agar with Lecithin and Polysorbate 80). The plates then are incubated for $24\pm2$ hours, and the colonies are counted for the number of survivors and the calculation of percent or log reduction. The control count (numbers control) is determined by conducting the procedure as described above with the exception that deionized water is used in place of the test composition. The plate counts are converted to cfu/ml for the numbers control and samples, respectively, by standard microbiological methods.

The log reduction is calculated using the formula

$$\text{Log reduction} = \log_{10}(\text{numbers controlled}) - \log_{10}(\text{test sample survivors}).$$

The following table correlates percent reduction in bacteria population to log reduction:

| % Reduction | Log Reduction |
|---|---|
| 90 | 1 |
| 99 | 2 |
| 99.9 | 3 |
| 99.99 | 4 |
| 99.999 | 5 |

b) Antiviral Residual Efficacy Test

References: S.A. Sattar, Standard Test Method for Determining the Virus-Eliminating Effectiveness of Liquid Hygienic Handwash Agents Using the Finger-pads of Adult Volunteers, Annual Book of ASTM Standards. Designation E1838-96, referred to as "Sattar I"; and S.A. Sattar et al., Chemical Disinfection to Interrupt Transfer of Rhinovirus Type 14 from Environmental Surfaces to Hands, Applied and Environmental Microbiology, Vol. 59, No. 5, May, 1993, pp. 1579-1585, referred to as "Sattar II."

The method used to determine the Antiviral Index of the present invention is a modification of that described in Sattar I, a test for the virucidal activity of liquid hand washes (rinse-off products). The method is modified in this case to provide reliable data for leave-on products.

Modifications of Sattar I include the product being delivered directly to the skin as described below, virus inoculation of the fingerpads as described below, and viral recovery using ten-cycle washing. The inoculated skin site then is completely decontaminated by treating the area with 70% dilution of ethanol in water.

Procedure:

Ten-minute Test:
Subjects (5 per test product) initially wash their hands with a nonmedicated soap, rinse the hands, and allow the hands to dry.

The hands then are treated with 70% ethanol and air dried.

Test product (1.0 ml) is applied to the hands, except for the thumbs, and allowed to dry.

About 10 minutes ($\pm30$ seconds), after product application, 10 ul of a Rhinovirus 14 suspension (ATCC VR-284, approximately $1\times10^6$ PFU (plaque-forming units)/ml) is topically applied using a micropipette to various sites on the hand within a designated skin surface area known as fingerpads. At this time, a solution of rhinovirus also is applied to the untreated thumb in a similar manner.

After a dry-down period of 7-10 minutes, the virus then is eluted from each of the various skin sites with 1 ml of eluent (Earle's Balanced Salt Solution (EBSS) with 25% Fetal Bovine Serum (FBS)+1% pen-strep-glutamate), washing 10 times per site.

The inoculated skin site then is completely decontaminated by rinsing the area with 70% ethanol. Viral titers are determined using standard techniques, i.e., plaque assays or $\text{TCID}_{50}$ (Tissue Culture Infectious Dose).

One-hour test:

Subjects are allowed to resume normal activities (with the exception of washing their hands) between the 1-hour and 3-hour timepoints. After one hour, a rhinovirus suspension is applied to and eluted from designated sites on the fingerpads exactly as described in above for the 10-minute test.

### Example 1

The following antiviral composition, which is capable of reducing skin pH, was prepared and applied to the fingerpads of human volunteers:

| Sample 2 | |
|---|---|
| **Material** | **percent (by weight)** |
| Ethanol | 70.0 |
| Deionized water | 19.8 |
| ULTREZ 20[1] | 1.0 |
| Isopropyl Palmitate | 1.0. |
| Mineral oil | 1.0 |
| DC 200 silicone fluid | 1.0 |
| Cetyl alcohol | 1.0 |
| Citric-acid | 2.0 |
| Malic acid | 2.0 |
| GERMABEN II[2] | 1.0 |
| Triethanolamine | 0.05 |
| | 100.0 |
| [1] Acrylate/C10-30 Alkyl Acrylate Crosspolymer; [2] Preservative containing propylene glycol, diazolidinyl urea, methylparaben, and propylparaben. | |

The pH of Sample 2 was 3.1.

In the test, Sample 2 was applied to the fingerpads of all fingers, except the thumbs, of eight volunteers. The thumbs were control sites. The volunteers were divided into fours groups of two each. Each group I-IV then was challenged at a predetermined time with rhinovirus titer on all the fingerpads of each hand to determine the time-dependent efficacy of the test composition. At the time appropriate for each group, the skin pH of the fingerpads also was measured to determine the time course of skin pH in response to the test composition. The predetermined test time for rhinoviral challenge and skin pH measurement for each group I-IV were 5 minutes, 1 hour, 2 hours, and 4 hours, respectively. The following table summarizes the average log (rhinoviral titer inoculum), average skin pH, and average log (rhinoviral titer recovered) from the test fingerpads. of the volunteers in the study, organized by group.

| Group | Initial skin pH after application (average) | Skin pH at test time (average) | Log [Inoculum Titer] (average) | Log [Recovered Titer] (average) |
|---|---|---|---|---|
| I | 3.0 | 3.0 | 3.9 | 0.23 |
| II | 2.8 | 3.4 | 4.0 | 0.23 |
| III | 3.0 | 3.8 | 3.8 | 0.23 |
| IV | 3.0 | 3.8 | 4.3 | 0.23 |

The data for each group (i.e., different time points) shows that the average recovered rhinoviral titer is less than 1 virus particle, or below the detection limit of the test. This data illustrates the efficacy of the present method after 4 hours and further demonstrates that a skin pH of less than about 4 is effective at completely eliminating a virus challenge.

### Examples 2

The clean fingerpads of test subjects were treated with the following composition. Baseline skin pH readings were measured from the fingerpads prior to treatment with the compositions. Skin pH measurements also were taken immediately after the composition dried on the fingerpads.

Immediately after treatment of the fingerpads with the composition, Rhinovirus 14 at a titer of 1.4 x $10^4$ pfu (plaque forming units) was applied to the finger-pads. The virus was dried on the fingerpads for 10 minutes, then the fingerpads were rinsed with a viral recovery broth containing 75% EBSS and 25% FBS with 1X antibiotics. The sample was diluted serially in viral recovery broth and plated onto H1-HeLa cells. Titers were assayed as per the plaque assay. Complete inactivation of Rhinovirus 14 was achieved with the acid-containing composition resulting in a 4 log reduction.

| Sample | Composition (by wt%) | Solution pH | Viral Log 10 Reduction 30 seconds | % Hands with Virus |
|---|---|---|---|---|
| A | 2% citric acid, 2% malic acid, 70% ETOH, 1% polyacrylic acid | 3.10 | 4 log | 0 |

### Example 3

Example 7 demonstrated that a synergism exists between polyacrylic acid and ethanol, which results in suppression of skin pH and antiviral efficacy. The following compositions were prepared to examine the effectiveness of polycarboxylic acid blends and a single polycarboxylic acid composition, each in combination with polyacrylic acid and ethanol, on antiviral efficacy. A preferred antiviral composition contains the least amount of organic acid required to demonstrate a persistent antiviral efficacy.

The compositions were applied to the fingerpads of clean hands. After the indicated times, about $10^3$ to $10^4$ pfu of Rhinovirus 39 was applied to the hands and allowed to dry for 10 minutes. The virus was recovered by rinsing the hands with viral recovery broth. The samples then were diluted serially in viral recovery broth and plated on HI-HeLa cells. Viral titers were determined by plaque assay. The percentage of hands that were positive for rhinovirus is summarized below.

| Composition (by wt%) | Time | % of Hands Positive for Rhinovirus |
|---|---|---|
| 70% ethanol | 15 min. | 100% |
| 1% citric acid/1% malic acid/10% ethanol/water | 1hr. | 100% |
| 1% polyacrylic acid/4% citric acid/70% ethanol/water | 4 hrs. | 91% |
| 1% polyacrylic acid/1% citric acid/1% malic acid/70% ethanol/water | 4 hrs. | 0% |

A composition containing 70% ethanol alone was not effective as an antiviral composition. Citric acid (1%) and malic acid (1%) lost effectiveness against rhinovirus after one hour because 100% of the hands were found to be positive for rhinovirus. In contrast, when a composition containing 1% citric and 1% malic acids are applied to the hands in combination with polyacrylic acid and 70% ethanol, no virus was detected on the hands after four hours, A single acid (4% citric acid) in combination with a polyacrylic acid and ethanol was less effective against rhinovirus because 91% of hands were found to be positive for rhinovirus after four hours.

This data demonstrates that using a polyacrylic acid and ethanol allows the use of a lower concentration of polycarboxylic acid to achieve a desired antiviral efficacy.

### Example 4

The use of a polyacrylic acid and ethanol in a composition suppresses skin pH to a value below the solution pH, as demonstrated in Example 7. To test whether antiviral compositions containing citric acid, malic acid, polyacrylic acid, and ethanol can be buffered to a higher solution pH and still provide a skin pH at or below pH 4 to obtain a persistent antiviral activity, the following compositions were prepared.

| Sample | Composition (by wt%) | Solution pH | Skin pH Initial | Skin pH 4 hrs. | Viral Reduction |
|---|---|---|---|---|---|
| A | 1% ULTREZ 20/2% citric acid/2% malic acid/70% ethanol | 3.2 | 2.9 _ | 3.7 | >3 $\log_{10}$ |
| B | 1% ULTREZ 20/2% citric acid/2% malic acid/70% ethanol | 4.34 | 3.4 | 3.7 | >3 $\log_{10}$ |
| C | 1% ULTREZ 20/2% citric acid/2% malic acid/70% ethanol | 4.65 | 3.6 | 3.8 | >3 $\log_{10}$ |

The compositions (1.8 mL) were applied to the thumb, index, and middle fingers of clean hands. Skin pH readings were measured prior to treatment (baseline), immediately after the fingers were dry, and again after four hours. The average of the skin pH values are plotted above.

Initial skin pH of skin treated with Samples A-C were suppressed to between pH 2.9 and 3.6, wherein the lower the solution pH, the lower the initial skin pH. However, after four hours, the skin pH for all three compositions was about pH 3.7. Consistent with previous examples, solution pH did not predict subsequent skin pH.

The viral efficacy of Samples A-C against Rhinovirus 39 also was tested. A viral load of about $10^3$ pfu was spread over the thumb, index, and middle fingers of each treated hand and allowed to dry for 10 minutes. The fingers then were rinsed with viral recovery broth and samples were diluted serially and plated on H1-HeLa cells. Viral titers were.measured using the plaque assay. No virus was recovered from any of the hands indicating that all three Samples A-C have antiviral efficacy.

This data demonstrates than when citric acid and malic acid are utilized in a composition in combination with a polyacrylic acid and ethanol, the pH of the solution can be buffered to a higher, e.g., milder and safer, pH for application to the skin, while still retaining an ability to suppress skin pH and exhibit antiviral activity.

The antimicrobial compositions of the present invention have several practical end uses, including mouthwashes, surgical scrubs, body splashes, antiseptics, disinfectants, hand sanitizer gels, deodorants, dental care additives, and similar personal care products. Additional types of compositions include foamed compositions, such as creams, mousses, and the like, and compositions containing organic and inorganic filler materials, such as emulsions, lotions, creams, pastes, and the like. The compositions further can be used as an antimicrobial for hard surfaces, for example, sinks and countertops in hospitals, food service areas, and meat processing plants. The present antimicrobial compositions can be manufactured as dilute ready-to-use compositions, or as concentrates that are diluted prior to use.

The present invention, therefore, encompasses applying an effective amount of the antimicrobial cleansing compositions of the present invention onto nonskin surfaces, such as household surfaces, e.g., countertops, kitchen surfaces, food preparing surfaces (cutting board, dishes, pots and pans, and the like); major household appliances, e.g., refrigerators, freezers, washing machines, automatic dryers, ovens, microwave ovens, and dishwashers; cabinets; walls; floors; bathroom surfaces, shower curtains, garbage cans, and/or recycling bins, and the like.

The compositions also can be incorporated into a web material to provide an antimicrobial wiping article. The wiping article can be used to clean and sanitize animate or inanimate surfaces.

In one embodiment of the present invention, a person who either (a) is suffering from a rhinovirus cold, or is likely to be exposed to other individuals suffering from rhinovirus colds, or (b) is suffering from a rotaviral infection, or is likely to be exposed to other individuals suffering from a rotaviral infection, can apply a present antimicrobial composition to his or her hands. This application kills bacteria and inactivates rhinovirus, rotavirus, and other nonenveloped virus particles present on the hands. The applied composition, either rinsed off or allowed to remain on the hands, provides a persistent antiviral activity. Nonenveloped viruses, like rhinovirus and rotavirus particles, therefore, are not transmitted to noninfected individuals via hand-to-hand transmission. The amount of the composition applied, the frequency of application, and the period of use will vary depending upon the level of disinfection desired, e.g., the degree of microbial contamination and/or skin soiling.

The present antimicrobial compositions provide the advantages of a broad spectrum kill of Gram positive and Gram negative bacteria, and a broad spectrum viral .control, in short contact times. The short contact time for a substantial log reduction of bacteria is important in view of the typical 15 to 60 second time frame used to sanitize the skin and inanimate surfaces. The composition also imparts a persistent antiviral activity to the contacted surface. The present compositions are effective in short contact time because of the synergistic effect provided by the combination of a disinfecting alcohol and an organic acid.

Obviously, many modifications and variations of the invention as hereinbefore set forth can be made without departing from the spirit and scope thereof, and, therefore, only such limitations should be imposed as are indicated by the appended claims.

**Claims**

1. A method of reducing and/or inactivating bacteria and a virus population on a hard, inanimate surface comprising contacting the surface with a composition for 30 seconds to achieve a log reduction of at least 2 against S. *aureus,* a log reduction of at least 2.5 against E. *coli,* and a log reduction of at least 4 against a nonevenloped virus, said composition comprising:

   (a) 25 to 75 % by weight of a disinfecting alcohol, comprising one or more $C_{1-6}$ alcohol;
   (b) 0.05 to 6 % by weight of an organic acid, comprising

      (i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
      (ii) a polymeric acid containing a plurality of carboxyl groups; and

   (c) water,

   wherein the composition has a pH of 5 or less at 25°C
   and optionally rinsing the composition from the surface.

2. Use of a composition for the manufacture of a medicament for therapeutic reduction and/or inactivation of bacteria and a virus population on a skin of a mammal comprising contacting the surface with a composition for 30 seconds to achieve a log reduction of at least 2 against S. *aureus,* a log reduction of at least 2.5 against E. *coli,* and a log reduction of at least 4 against a nonevenloped virus, said composition comprising:

   (a) 25 to 75 % by weight of a disinfecting alcohol, comprising one or more $C_{1-6}$ alcohol;
   (b) 0.05 to 6 % by weight of an organic acid, comprising

      (i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid malic acid, maleic acid, citric acid, aconitic acid and mixtures thereof and
      (ii) a polymeric acid containing a plurality of carboxyl groups; and

   (c) water,

   wherein the composition has a pH of 5 or less at 25°C
   and the composition lowers the pH of the skin to less than 4 after drying on the skin.

3. The method of claim 1 or use of claim 2, wherein the virus is an acid-labile virus or a noneveloped virus.

4. The method or use of claim 1 to 3, wherein the acid labile virus comprises a rhinovirus serotype or a rotavirus serotype.

5. The method or use of claim 1 to 4, wherein the polymeric acids has a molecular weight of 500 to 10,000,000 g(mol, wherein the polymeric acid is water soluble or water dispersible.

6. The method or use of claim 1 to 5, wherein the polymeric acids are selected from the group consisting of a polymeric carboxylic acid, a homopolymer or a copolymer of acrylic acid and mixtures thereof.

7. The method or use of claim 1 to 6, wherein the polycarboxylic acids comprise at least two of citric acid, malic acid, tartaric acid; and the polymeric caroxylic acid comprises a homopolymer or a copolymer of acrylic acid or methacrylic acid.

8. The use of claim 2 to 7, wherein the skin of a mammal has a skin pH of less than 4 four hours after contact and wherein the composition imparts a log reduction of at least 3 against a noneveloped virus at least four hours after contact.

9. The method or use of claim 1 to 8 wherein the composition further comprises one or more of

(a) 0.1 to 30 % by weight of a polyhydric solvent selected from the group consisting of a diol, a triol, and mixtures thereof; (b) 0.1 to 30 % by weight of a hydrotrope; (c) 0.1 to 3 % by weight of a gelling agent, wherein the gelling agent is selected from the group consisting of cellulose, a cellulose derivative, guar, a guar derivative, algin, an algin derivative, a water insoluble $C_8$-$C_{20}$-alcohol, carrageenen, a smectite clay, a polyquaternium compound, and mixtures thereof.

10. The method or use of claim 1 to 9 wherein the composition is free of an anionic, cationic, and ampholytic surfactant,

11. The method or use of claim 1 to 10 whereinrhinoviruses, picornaviruses, adenoviruses, rotaviruses, herpes viruses, respiratory synctial viruses, coronaviruses, enterovirusesm, and similar pathogenic viruses are inactivated.

12. An antimicrobial composition, comprising:

(a) 25 to 75 % by weight of a disinfecting alcohol, comprising one or more $C_{1-6}$ alcohol;
(b) 0.05 to 6 % by weight of an organic acid, comprising

(i) more than one polycarboxylic acid, selected from the group consisting of malonic acid, succinc acid, glutaric acid, adipic acid, pimeelic acid, suberic acid, azealic acid, sebacic acid, fumaric acid, maleic acid, tartaric acid, malic acid, maleic acid, citric acid, aconitic acid and mixtures thereof and
(ii) a polymeric acid containing a plurality of carboxyl groups; and

(c) water,
(d) optionally 0.01 to 5 % by weight of a gelling agent

wherein the composition has a pH of 5 or less at 25°C.

**Patentansprüche**

1. Verfahren zur Minimierung und/oder Inaktivierung von Bakterien- und Viruspopulationen auf harten Oberflächen, bei dem die Oberfläche für 30 Sekunden mit einer Zusammensetzung kontaktiert wird, um eine Logminimierung von mindestens 2 gegen S. aureus, eine Logminimierung von mindestens 2,5 gegen E. coli und eine Logminimierung von mindestens 4 gegen nicht umhüllte Viren erreicht wird, wobei die Zusammensetzung:

(a) 25 bis 75 Gew.-% eines desinfizierenden Alkohols ausgewählt aus $C_{1-6}$ Alkoholen;
(b) 0,05 bis 6 Gew.-% einer organischen Säure, umfassend

(i) mehr als eine Polycarbonsäure, ausgewählt aus der Gruppe Malonsäure, Bernsteinsäure, Glutarsäure, Adipinsäure, Pimelinsäure, Korksäure, Acelainsäure, Sebacinsäure, Fumarsäure, Maleinsäure, Weinsäure, Äpfelsäure, Maleinsäure, Zitronensäure, Aconitinsäure sowie Mischungen davon und
(ii) eine polymere Säure, umfassend eine Vielzahl von Carboxylgruppen; und

(c) Wasser,

wobei die Zusammensetzung einen pH-Wert von 5 oder weniger bei 25°C aufweist,
und optional Abspülen der Zusammensetzung von der Oberfläche.

2. Verwendung einer Zusammensetzung für die Herstellung eines Medikaments für die therapeutische Minimierung und/oder Inaktivierung von Bakterien- und Viruspopulationen auf der Haut eines Lebewesens, umfassend in Kontakt bringen der Oberfläche für 30 Sekunden, um eine Logminimierung von mindestens 2 gegen S. aureus, eine Logminimierung von mindestens 2,5 gegen E. coli und eine Logminimierung von mindestens 4 gegen nicht umhüllte Viren erreicht wird, wobei die Zusammensetzung:

(a) 25 bis 75 Gew.-% eines desinfizierenden Alkohols ausgewählt aus $C_{1-6}$ Alkoholen;
(b) 0,05 bis 6 Gew.-% einer organischen Säure, umfassend

(i) mehr als eine Polycarbonsäure, ausgewählt aus der Gruppe Malonsäure, Bernsteinsäure, Glutarsäure, Adipinsäure, Pimelinsäure, Korksäure, Acelainsäure, Sebacinsäure, Fumarsäure, Maleinsäure, Weinsäure, Äpfelsäure, Maleinsäure, Zitronensäure, Aconitinsäure sowie Mischungen davon und
(ii) eine polymere Säure, umfassend eine Vielzahl von Carboxylgruppen; und

(c) Wasser,

wobei die Zusammensetzung einen pH-Wert von 5 oder weniger bei 25°C aufweist,
und die Zusammensetzung den pH-Wert der Haut auf weniger als 4 verringert, nachdem sie auf der Haut getrocknet ist.

3. Verfahren gemäß Anspruch 1 oder Verwendung gemäß Anspruch 2 worin der Virus ein Säure-labiler Virus oder ein nicht umhüllter Virus ist.

4. Verfahren oder Verwendung nach einem der Ansprüche 1 - 3, wobei der Säure-labile Virus einen Rhinovirus Serotypen oder einen Rotavirus Serotypen umfasst.

5. Verfahren oder Verwendung nach einem der Ansprüche 1 - 4, wobei die polymere Säure eine Molmasse von 500 - 10.000.000 g/mol aufweist und worin die polymere Säure wasserlöslich oder wasserdispergierbar ist.

6. Verfahren oder Verwendung nach einem der Ansprüche 1 - 5, wobei die polymere Säure ausgewählt ist aus der Gruppe der polymeren Carboxysäuren, der Homo- oder Copolymeren von Acrylsäure sowie Mischungen davon.

7. Verfahren oder Verwendung nach einem der Ansprüche 1 - 6, worin die Polycarbonsäuren mindestens zwei Vertreter aus der Gruppe Zitronensäure, Äpfelsäure, Weinsäure umfassen und die polymeren Carboxysäuren ein Homopolymer oder ein Copolymer von Acrylsäure oder Methacrylsäure umfassen.

8. Verwendung nach einem der Ansprüche 2 - 7, wobei die Haut einen Haut-pH-Wert von weniger als 4, 4 Stunden nach Kontakt aufweist, und wobei die Zusammensetzung eine Logminimierung von mindestens 3 gegen einen nicht umhüllten Virus, mindestens 4 Stunden nach Kontakt erreicht.

9. Verfahren oder Verwendung nach einem der Ansprüche 1 - 8, wobei die Zusammensetzung weiter einen oder mehrere folgender Inhaltsstoffe enthält:

(a) 0,1 - 30 Gew.-% eines polyhydridischen Lösungsmittels ausgewählt aus der Gruppe der Diole, der Triole und deren Mischungen,
(b) 0,1 - 30 Gew.-% eines Hydrotops,
(c) 0,1 - 3 Gew.-% eines Gelbildners, wobei der Gelbildner ausgewählt ist aus der Gruppe Cellulose, Cellulose-Derivate, Guar, Guar-Derivate, Algin, Algin-Derivate, wasserunlösliche $C_8$-$C_{20}$-Alkohole, Carageenan, Smectidtone, Polyquaternium-Verbindungen sowie deren Mischungen.

10. Verfahren oder Verwendung nach einem der Ansprüche 1 - 9, wobei die Zusammensetzung frei von anionischen, kationischen und amphoteren Tensiden ist.

11. Verfahren oder Verwendung nach einem der Ansprüche 1 - 10, wobei Rhinoviren Picornaviren, Adenoviren, Rotaviren, Herpesviren, synktiale Resperationsviren, Koronaviren, Enteroviren und ähnliche patugenische Viren inaktiviert werden.

12. Antimikrobielle Zusammensetzung, enthaltend:

(a) 25 bis 75 Gew.-% eines desinfizierenden Alkohols ausgewählt aus $C_{1-6}$ Alkoholen;
(b) 0,05 bis 6 Gew.-% einer organischen Säure, umfassend

(i) mehr als eine Polycarbonsäure, ausgewählt aus der Gruppe Malonsäure, Bernsteinsäure, Glutarsäure, Adipinsäure, Pimelinsäure, Korksäure, Acelainsäure, Sebacinsäure, Furmarsäure, Maleinsäure, Weinsäure, Äpfelsäure, Maleinsäure, Zitronensäure, Akonitinsäure sowie Mischungen davon und
(ii) eine polymere Säure, umfassend eine Vielzahl von Carboxylgruppen; und

(c) Wasser,
(d) gegebenenfalls 0,01 - 5 Gew.-% eines Gelbildners,

wobei die Zusammensetzung einen pH von 5 oder weniger bei 25°C aufweist.

**Revendications**

1. Procédé de réduction et/ou d'inactivation de bactéries et d'une population virale sur une surface inerte dure, comprenant la mise en contact de la surface avec une composition pendant 30 secondes pour obtenir une réduction logarithmique d'au moins 2 vis-à-vis de S. *aureus,* une réduction logarithmique d'au moins 2,5 vis-à-vis de E. *coli*, et une réduction logarithmique d'au moins 4 vis-à-vis d'un virus non enveloppé, ladite composition comprenant :

   (a) à concurrence de 25 à 75 % en poids, un alcool désinfectant, comprenant un ou plusieurs alcools en $C_1$-$C_6$;
   (b) à concurrence de 0,05 à 6 % en poids, un acide organique, comprenant :

   (i) plus d'un acide polycarboxylique choisi parmi le groupe constitué par l'acide malonique, l'acide succinique, l'acide glutarique, l'acide adipique, l'acide pimélique, l'acide subérique, l'acide azélaïque, l'acide sébacique, l'acide fumarique, l'acide maléique, l'acide tartrique, l'acide malique, l'acide maléique, l'acide citrique, l'acide aconitique et leurs mélanges, et
   (ii) un acide polymérique contenant plusieurs groupes carboxyle ; et

   (c) de l'eau ;

   la composition possédant un pH de 5 ou moins à 25°C ;
   et, de manière facultative, éliminer la composition de la surface par rinçage.

2. Utilisation d'une composition pour la préparation d'un médicament destiné à la réduction et/ou à l'inactivation thérapeutique de bactéries et d'une population virale sur la peau d'un mammifère, comprenant la mise en contact de la surface avec une composition pendant 30 secondes pour obtenir une réduction logarithmique d'au moins 2 vis-à-vis de S. *aureus,* une réduction logarithmique d'au moins 2,5 vis-à-vis de E. *coli,* et une réduction logarithmique d'au moins 4 vis-à-vis d'un virus non enveloppé, ladite composition comprenant :

   (a) à concurrence de 25 à 75 % en poids, un alcool désinfectant, comprenant un ou plusieurs alcools en $C_1$-$C_6$ ;
   (b) à concurrence de 0,05 à 6 % en poids, un acide organique, comprenant :

   (i) plus d'un acide polycarboxylique choisi parmi le groupe constitué par l'acide malonique, l'acide succinique, l'acide glutarique, l'acide adipique, l'acide pimélique, l'acide subérique, l'acide azélaïque, l'acide sébacique, l'acide fumarique, l'acide maléique, l'acide tartrique, l'acide malique, l'acide maléique, l'acide citrique, l'acide aconitique et leurs mélanges, et
   (ii) un acide polymérique contenant plusieurs groupes carboxyle ; et

   (c) de l'eau ;

   la composition possédant un pH de 5 ou moins à 25°C ;
   et la composition diminuant le pH de la peau jusqu'à une valeur inférieure à 4 après le séchage sur la peau.

3. Procédé selon la revendication 1 ou utilisation selon la revendication 2, dans lequel le virus est un virus labile en présence d'un acide ou un virus non enveloppé.

4. Procédé ou utilisation selon l'une quelconque des revendications 1 à 3, dans lequel le virus labile en présence d'un acide comprend un sérotype de rhinovirus ou un sérotype de rotavirus.

5. Procédé ou utilisation selon l'une quelconque des revendications 1 à 4, dans lequel les acides polymériques possèdent un poids moléculaire de 500 à 10.000.000 g/mol, l'acide polymérique étant soluble dans l'eau ou apte à être dispersé dans l'eau.

6. Procédé ou utilisation selon l'une quelconque des revendications 1 à 5, dans lequel les acides polymériques sont choisis parmi le groupe constitué par un acide carboxylique polymérique, un homopolymère ou un copolymère d'acide acrylique, ainsi que leurs mélanges.

**7.** Procédé ou utilisation selon l'une quelconque des revendications 1 à 6, dans lequel les acides polycarboxyliques comprennent au moins deux acides choisis parmi l'acide citrique, l'acide malique, l'acide tartrique, et l'acide carboxylique polymérique comprend un homopolymère ou un copolymère d'acide acrylique ou d'acide méthacrylique.

**8.** Utilisation selon l'une quelconque des revendications 2 à 7, dans laquelle la peau d'un mammifère possède un pH cutané inférieur à 4, quatre heures après la mise en contact et dans laquelle la composition confère une réduction logarithmique d'au moins 3 vis-à-vis d'un virus non enveloppé au moins quatre heures après la mise en contact.

**9.** Procédé ou utilisation selon l'une quelconque des revendications 1 à 8, dans lequel la composition comprend en outre un ou plusieurs éléments choisis parmi : (a) à concurrence de 0,1 à 30 % en poids, un solvant polyhydrique choisi parmi le groupe constitué par un diol, un triol et leurs mélanges ; (b) à concurrence de 0,1 à 30 % en poids, un hydrotrope ; (c) à concurrence de 0,1 à 3 % en poids, un agent gélifiant, l'agent gélifiant étant choisi parmi le groupe constitué par la cellulose, un dérivé de la cellulose, le guar, un dérivé du guar, l'algine, un dérivé d'algine, un alcool en $C_8$-$C_{20}$ insoluble dans l'eau, le carraghénane, une argile de smectite, un composé de polyquaternium, et leurs mélanges.

**10.** Procédé ou utilisation selon l'une quelconque des revendications 1 à 9, dans lequel la composition est exempte d'un agent tensioactif anionique, cationique et ampholytique.

**11.** Procédé ou utilisation selon l'une quelconque des revendications 1 à 10, dans lequel des rhinovirus, des picornavirus, des adénovirus, des rotavirus, des herpèsvirus, des virus respiratoires synctiaux, des coronavirus, des entérovirus et des virus pathogènes similaires sont inactivés.

**12.** Composition antimicrobienne, comprenant :

(a) à concurrence de 25 à 75 % en poids, un alcool désinfectant, comprenant un ou plusieurs alcools en $C_1$-$C_6$ ;
(b) à concurrence de 0,05 à 6 % en poids, un acide organique, comprenant :

(i) plus d'un acide polycarboxylique choisi parmi le groupe constitué par l'acide malonique, l'acide succinique, l'acide glutarique, l'acide adipique, l'acide pimélique, l'acide subérique, l'acide azélaïque, l'acide sébacique, l'acide fumarique, l'acide maléique, l'acide tartrique, l'acide malique, l'acide maléique, l'acide citrique, l'acide aconitique et leurs mélanges, et
(ii) un acide polymérique contenant plusieurs groupes carboxyle ; et

(c) de l'eau ;
(d) de manière facultative, à concurrence de 0,01 à 5 % en poids, un agent gélifiant,

la composition possédant un pH de 5 ou moins à 25 °C.

## REFERENCES CITED IN THE DESCRIPTION

**Patent documents cited in the description**

- US 63446404 P
- US 63448304 P
- US 6107261 A
- US 6136771 A
- US 6110908 A
- US 5776430 A
- EP 0604848 A
- US 5968539 A
- US 6106851 A

- US 6113933 A
- US 4767788 A
- US 4975217 A
- US 20020098159 A
- US 6034133 A
- US 6294186 B
- US 6436885 B
- WO 9746218 A
- WO 9606152 A

**Non-patent literature cited in the description**

- Fundamental Virology. Lippincott Williams & Wilkins, 2001
- **Hayden et al.** *Antimicrobial Agents and Chemotherapy,* 1984, vol. 26, 928-929
- **Hayden et al.** *Journal of Infectious Diseases,* 1985, vol. 152, 493-497

- Standard Test Method for Determining the Virus-Eliminating Effectiveness of Liquid Hygienic Handwash Agents Using the Finger-pads of Adult Volunteers. **S.A. Sattar.** Annual Book of ASTM Standards. Designation E1838-96
- **S.A. Sattar et al.** Chemical Disinfection to Interrupt Transfer of Rhinovirus Type 14 from Environmental Surfaces to Hands. *Applied and Environmental Microbiology,* May 1993, vol. 59 (5), 1579-1585